# EUROPEAN PATENT APPLICATION

(11) **EP 4 417 306 A1**
(43) Date of publication of application: **21.08.2024**
(21) Application number: 22880371.4
(22) Date of filing: 13.10.2022
(51) Int. Cl.: B01J 29/70, B01J 29/08, B01J 29/18, C10G 25/03

(54) **OLEFIN CONVERSION CATALYST, PREPARATION METHOD THEREFOR AND APPLICATION THEREOF**

(30) Priority: 13.10.2021 CN 202111195240
(71) Applicant: Shanghai Research Institute of Petrochemical Technology, SINOPEC, Shanghai 201208 (CN)
(72) Inventor: LI, Wei, Shanghai 201208 (CN); ZHOU, Yaxin, Shanghai 201208 (CN); WU, Lihin, Shanghai 201208 (CN); HOU, Min, Shanghai 201208 (CN)
(74) Representative: karo IP
(86) International application number: PCT/CN2022/125061
(87) International publication number: WO 2023/061435

(57) **Abstract**

An olefin conversion catalyst and its preparation method and application are disclosed. The catalyst comprises the following components, in parts by mass: a) 50-90 parts of a molecular sieve with a structure of twelve-membered ring and above; b) calculated as oxide, 0.1-10 parts of an added component selected from Group IA metal elements, Group IIA metal elements, or a combination thereof; c) calculated as oxide, 0.1-10 parts of a modifying component selected from silicon, germanium, bismuth, tin, boron, gallium or a combination thereof; and d) 10-49 parts of a support component. When the catalyst is used to convert a small amount of olefins in an aromatic distillate oil, it has the characteristics of high activity and stability, long life and effective utilization of olefins.

## Description

### Technical field

This application relates to the field of catalysts, and specifically relates to an olefin conversion catalyst, its preparation method and application.

### Background art

Benzenes products, which have the largest production capacity among petroleum aromatics, mainly come from aromatics complex units, ethylene units and refining units. A certain amount of olefin compounds are inevitably generated among aromatic hydrocarbon mixtures from the intermediate production process. The chemical properties of these olefin compounds are relatively active, and they are easy to produce new components, which may have a great effect on product quality and also cause trouble for the routine operation of downstream equipment. In order to obtain qualified aromatic products such as para-xylene and ensure the no problem to subsequent processes, trace olefin compounds need to be removed after the reforming, aromatic extraction, isomerization, and toluene disproportionation processes. According to the current technology, the above olefins are removed as a bad resource, but they are not effectively utilized.

As a traditional aromatic hydrocarbon de-alkenization refining agent, white clay has low activity, short life, large dosage, cannot be regenerated, and needs to be replaced frequently, which is not only detrimental to safe production, but also waste white clay pollutes the environment and requires high disposal costs. To this end, a molecular sieve olefin removal refining technology has been developed, which uses a molecular sieve catalyst with more active centers to replace industrial white clay, and utilizes its relatively large specific surface area and acid content to achieve a long single-cycle service life and total service life, and remove olefins as impurities. However, the catalyst needs to be regenerated ex-situ after each deactivation. During the molecular sieve regeneration process, due to high-temperature carbon burning, the activity of the molecular sieve catalyst is easily reduced. During the regeneration process, a large amount of olefin products generate carbon dioxide and water and are discharged into the atmosphere, which wastes a lot of resources.

CN102008976A, CN103041841B, CN102039160B and CN104907090A all use molecular sieve as the main active component of the olefin removal catalyst, which are improved through various methods to be suitable for the olefin removal reaction process and prevent the catalyst from coking and deactivating too quickly. Although the catalyst life is relatively increased, the organic matter of the olefin products is more adsorbed and the life is not long. At the same time, due to the large amount of disassembly and assembly, the stable operation of the device is affected. Among them, the olefin removal catalyst disclosed in CN102008976A uses high silicon-to-aluminum ratio ReUSY molecular sieve as the main active component, mordenite as the second active component, and alumina as the binder. Macromolecule olefin impurities are not easy to diffuse in the catalyst, which leads to coking and deactivation of the catalyst active center. CN103041841B discloses an aromatic hydrocarbon non-hydrogenation olefin removal catalyst and a preparation method thereof, which uses waste catalytic cracking catalyst containing Y molecular sieve as raw materials to produce the aromatic hydrocarbon non-hydrogenation olefin removal catalyst. A waste catalytic cracking catalyst with a metal content of less than 5000 µg/g and alumina dry rubber powder are mixed and extruded into strips, and then calcined at 540°C for 4 hours to obtain an aromatic hydrocarbon non-hydrogenation olefin removal catalyst. This method only aims at rejuvenation the deactivated catalyst and reduces the catalyst cost by reducing the catalyst preparation cost, without improving the actual production cycle. The reformateate olefin removal catalyst introduced in CN102039160B uses 20 to 90 parts of a molecular sieve and 10 to 80 parts of at least one selected from SiO₂, Al₂O₃ or a mixture thereof, and one catalyst containing at least one metal selected from Mo, Zr, Nb or its oxide, at least one element selected from Cl, Br, S or its oxide, at least one element selected from F, P or its oxide, which effectively prolong the regeneration cycle of catalyst, but it cannot be regenerated because it contains Cl, Br, S, and F. Also, the activity is easily covered or blocked by coke, the life is short, and the olefin cannot be used after removal. CN104907090A introduces a catalytic reformate refining olefin removal catalyst and its preparation method, including 30% to 70% Al₂O₃ and 30% to 70% molecular sieve, with Al oxide as the active center, and is prepared by an impregnation method, the active center in the molecular sieve is easily blocked by coking, and after the olefin is removed, it cannot be used.

Another way of olefin removal is hydrogenation technology. Hydrogenation technology uses hydrogen to react with olefins to saturate double bonds to selectively remove olefins. However, it also inevitably hydrogenates unsaturated aromatic rings in some high-value aromatic hydrocarbons, generating irreversible non-aromatic hydrocarbons and reducing the production capacity of the entire device. For example, in CN1448474A, noble metals are used as active metal components. Due to the excessive hydrogenation performance, alkali metals and alkaline earth metals are used as additives to suppress excessive hydrogenation performance. However, the loss of aromatic hydrocarbons is high, reaching close to 0.5wt%, which directly increases the cost of aromatics processing. CN101260320A uses an eggshell-shaped mode to optimize metal distribution to reduce excessive hydrogenation performance, but there is still inevitable loss of aromatics. In CN108636399A, a hydrogenation method by non-noble metal is used to remove olefins, which not only consumes hydrogen, but also requires reduction and activation before the catalyst is used. It is difficult to apply in actual industry. At the same time, some aromatic hydrocarbons are inevitably lost during operation, and the activity is also difficult to maintain stable.

The above catalysts use different modification methods to conduct deolefination. The molecular sieve olefin removal method has a short life; the hydrogenation technology is costly and difficult to operate. In both methods, olefins cannot be fully utilized, resulting in an increase in the production cost of aromatics.

### Summary of the Invention

In order to solve the problems in the prior art that the olefin removal catalyst is easy to coke and deactivate, has poor stability, and has a short service life, the object of the present application is to provide an olefin conversion catalyst and its preparation method and application. When the catalyst is used to convert the olefins in the aromatic-rich reformate, it has the advantages of high activity stability, long life and effective utilization of olefins.

In order to achieve the above object, on one hand, the present application provides an olefin conversion catalyst, comprising the following components, in parts by mass:
a) 50-90 parts of a molecular sieve with a structure of twelve-membered ring and above;
b) calculated as oxide, 0.1-10 parts of an added component selected from Group IA metal elements, Group IIA metal elements, or a combination thereof;
c) calculated as oxide, 0.1-10 parts of a modifying component selected from silicon, germanium, bismuth, tin, boron, gallium or a combination thereof; and
d) 10-49 parts of a support component.

In another aspect, the present application provides a method for preparing the catalyst of the present application, comprising:
(1) shaping a mixture of the molecular sieve, the support and a precursor of the added component, drying and calcining it to obtain a shaped body; and
(2) loading the modifying component on the shaped body, drying and calcining it to obtain a catalyst.

In yet another aspect, the present application provides a method for converting olefins contained in an aromatic-rich distillate oil, comprising a step of contacting and reacting the aromatic-rich distillate oil containing olefins with the catalyst of the present application.

When the catalyst of the present application is used in the conversion process of a small amount of olefins in the aromatic-rich distillate oil, the olefins and aromatic hydrocarbons in the aromatic-rich feedstock can effectively converts the olefins into required aromatic components (such as C-8 aromatic hydrocarbons) through alkylation reaction, disproportionation reaction, transalkylation reaction or lightening reaction of heavy aromatic hydrocarbons, etc.; it not only reduces the content of olefins in the aromatic-rich distillate oil, but also increases the content of useful aromatic hydrocarbon components in the aromatic-rich distillate oil; it can also limits the generation of products with high boiling point; in addition, it greatly improves the activity stability and service life of the catalyst, which can increase the single-cycle life of the olefin conversion catalyst to more than 2-8 times the life of the current olefin removal catalyst.

### Description of Figures

The figures are used to provide a further understanding of the present application and constitute a part of the specification. They are used to explain the present application together with the following specific embodiments, but do not constitute a limitation of the present application. In the figures:

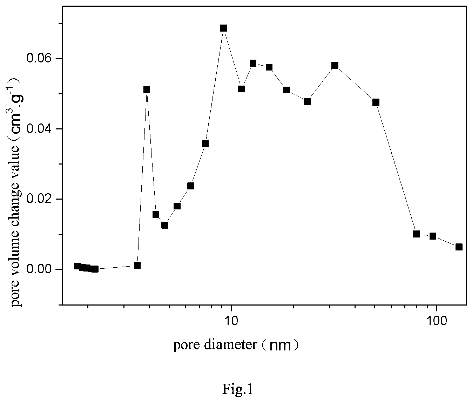

Figure 1 is the BET measurement and t-plot method calculation results of the mesopore distribution of the catalyst obtained in Example 1.

### Detailed Description of the Invention

Specific embodiments of the present application will be described in detail below with reference to the figures. It should be understood that the specific implementations described here are only used to illustrate and explain the present application, and are not intended to limit the present application.

Any specific numerical value disclosed herein (including the endpoints of a numerical range) is not limited to the precise value of the numerical value, but is to be understood to also encompass values close to the precise value, such as all possible values within ±5% of the precise value. Moreover, for the disclosed numerical range, one or more new numerical ranges can be obtained by any combination between the endpoint values of the range, between the endpoint value and the specific point value within the range, and between each specific point value; these new numerical ranges should also be deemed to be specifically disclosed herein.

Unless otherwise stated, the terms used herein have the same meanings as commonly understood by those skilled in the art. If a term is defined herein and its definition is different from the common understanding in the art, the definition herein shall prevail.

In the present application, the pore volume and pore size distribution of the molecular sieve and catalyst are measured using a liquid nitrogen adsorption and desorption instrument. The measurement method is as follows: conduct the test at the liquid nitrogen saturation temperature (77K), obtain N₂ isothermal adsorption-desorption curves through different relative pressures, and obtain pore volume and pore distribution by t-plot method; the relative pressure during the experiment is 0.001-0.995.

In the present application, the composition of the catalyst is determined by X-ray fluorescence spectroscopy (XRF).

In the present application, the micropore volume, mesopore volume and total pore volume are measured by the liquid nitrogen adsorption and desorption BET method and calculated by the t-plot method, wherein the total pore volume is calculated by the single point method at a relative pressure of 0.995.

In the present application, except for what is expressly stated, any matters or items not mentioned shall directly apply to those known in the art without any changes. Moreover, any embodiment described herein can be freely combined with one or more other embodiments described herein, and the technical solutions or technical ideas formed thereby are regarded as part of the original disclosure or original record of the present invention and shall not be regarded as new content that has not been disclosed or expected in this disclosure, unless those skilled in the art believe that the combination is obviously unreasonable.

All patent and non-patent literatures mentioned herein, including but not limited to textbooks and journal articles etc., are hereby incorporated by reference in their entirety.

In a first aspect, the present application provides an olefin conversion catalyst, comprising the following components in parts by mass:
a) 50-90 parts of a molecular sieve with a structure of twelve-membered ring and above;
b) calculated as oxide, 0.1-10 parts of an added component selected from Group IA metal elements, Group IIA metal elements, or a combination thereof;
c) calculated as oxide, 0.1-10 parts of a modifying component selected from silicon, germanium, bismuth, tin, boron, gallium or a combination thereof; and
d) 10-49 parts of a support component.

In the catalyst of the present application, the "added component" is introduced into the catalyst before the molecular sieve and support component are formed, and it can be detected through Scanning Electron Microscopy Energy Spectroscopy (SEM-EDS) that it is mainly distributed on the outer surface of molecular sieve grains. For example, the proportion of the content of the added component on the outer surface of the molecular sieve grains measured by SEM-EDS relative to the total content of the added component in the catalyst measured by the XRF method is more than 50%, such as 50-98%, 60-98% or 70-95%.

In the catalyst of the present application, the "modifying component" is introduced into the catalyst after the molecular sieve and support components are formed. For non-silica modifying component, through SEM-EDS characterization, it can be detected that the part distributed on the outer surface of the molecular sieve grains is relatively small relative to its total amount, indicating that it should mainly be distributed inside the molecular sieve grains. For example, the proportion of the content of the added component on the outer surface of the molecular sieve grains measured by SEM-EDS relative to the total content of the added component in the catalyst measured by the XRF method is below 50%, such as 10-45%, 10-40% or 10-35%.

Without being limited to a specific theory, it is believed that in the catalyst of the present application, the added component can prevent the colloid from adsorbing on the support and inhibit further reaction of the reaction product; and the modifying component has the function of capable of adjusting the pore channel diameter, and increasing the probability of producing C8 aromatic hydrocarbons from C9 aromatic hydrocarbons when the alkylation reaction product is generated.

In a preferred embodiment, the molecular sieve is selected from those having a twelve-membered or eighteen-membered ring structure, preferably selected from Y zeolite (such as USY ), β zeolite, MCM-22, MCM-56, SAPO-5, SAPO-37, SAPO-40, RZM-3 or a combination thereof. Further preferably, the zeolite is selected from Y, β , MCM-56, MCM-22 or a combination thereof. Particularly preferably, the SiO₂/Al₂O₃ molar ratio of the molecular sieve is 2 to 60, preferably 5 to 30, such as 8, 10, 12, 15, 18, 20, 22, 25 or 28.

In certain preferred embodiments, the molecular sieve is an ammonium-type molecular sieve. According to the present application, the term "ammonium-type molecular sieve" has the meaning commonly understood in the art, and specifically refers to a zeolite obtained by combining the acidic sites on the skeleton Al or other non-silicon elements in the ammonic molecular sieve , it can generally be obtained by using a synthetic solution containing ammonic ions during the synthesis process, ion-exchanging the molecular sieve with a solution containing ammonic ions after the synthesis, or combining ammonia gas with a hydrogen-type molecular sieve.

In a preferred embodiment, the support component is selected from or derived from alumina, alumina-containing clays, silica, or a combination thereof, preferably selected from or derived from alumina, kaolin, attapulgite, bentonite, diatomite, silica or a combination thereof, more preferably alumina.

In a preferred embodiment, the added component is selected from potassium, sodium, calcium, magnesium or a combination thereof, preferably magnesium.

In a preferred embodiment, the catalyst has the following pore distribution, characterized by nitrogen adsorption and desorption BET:
The pore volume of micropores with a diameter of greater than 0.5nm and less than 2.0nm accounts for 4-28% of the total pore volume, preferably 5-22%, such as 8%, 10%, 12%, 15%, 18% or 20%; the pore volume of mesopores with a diameter of 2 to 50 nm accounts for 6 to 50% of the total pore volume, preferably 10 to 40%, such as 15%, 20%, 25%, 30% or 35%.

In a further preferred embodiment, the ratio of the pore volume of the mesopores to the pore volume of the micropores of the catalyst is 0.5-8.0, preferably 0.8-4.5, such as 1.0, 1.5, 2.0, 2.5, 3.0, 3.5 or 4.0.

In the catalyst of the present application, the content of the added component in the catalyst is mainly combined with the molecular sieve, and active centers are distributed on the surface of the molecular sieve channels, so they are correlated with the molecular sieve content, and further are correlated with the ratio of the mesopore volume to the total pore volume of the molecular sieve. Preferably, the ratio of the mass of the added component calculated as oxide to the product of the mass of the molecular sieve and the ratio of the mesopore volume to the total pore volume of the catalyst is not greater than 0.50, preferably 0.08-0.35, such as 0.1, 0.12, 0.15, 0.18, 0.20, 0.22, 0.25, 0.28, 0.30 or 0.32.

In a preferred embodiment, the modifying component is a combination of silicon and at least one selected from tin, bismuth, germanium, gallium and boron, preferably a combination of silicon and tin. Preferably, calculated as oxide, the mass ratio of silicon to non-silicon modifying component (i.e., tin, bismuth, germanium, gallium, boron or a combination thereof) is 0.1-10.0:1, preferably 1.5-5.0:1, such as 2.0:1, 2.5:1, 3.0:1, 3.5:1, 4.0:1 or 4.5:1.

In a preferred embodiment of the catalyst, the molecular sieve is 55-80 parts, such as 58, 60, 65, 68, 70, 72 or 75 parts, in parts by mass.

In a preferred embodiment of the catalyst, the support component is 15-40 parts, such as 18, 20, 22, 25, 28, 30, 32, 35 or 38 parts, in parts by mass.

In a preferred embodiment of the catalyst, the added component is 1.0-8.0 parts, for example, 1.5, 2.0, 2.5, 3.0, 3.5, 4.0, 4.5, 5.0, 5.5, 6.0, 6.5, 7.0 or 7.5 parts, in parts by mass.

In a preferred embodiment of the catalyst, the modifying component is 1.0-8.0 parts, such as 1.5, 2.0, 2.5, 3.0, 3.5, 4.0, 4.5, 5.0, 5.5, 6.0, 6.5, 7.0 or 7.5 parts, in parts by mass.

In certain preferred embodiments, the total amount of the catalyst is 100 parts, in parts by mass.

In a second aspect, the present application provides a method for preparing an olefin conversion catalyst, including the following steps:
(1) shaping a mixture containing a molecular sieve with a structure of twelve-membered ring and above, a support and a precursor of added component, drying and calcining it to obtain a shaped body; and
(2) loading a precursor of modifying component on the shaped body, drying and optionally calcining it, to obtain a catalyst.

In the method of the present application, the molecular sieve, the support and the added component in step (1), and the modifying component in step (2) are as described in the first aspect of the present application, and will not be described again here.

In a preferred embodiment, as characterized by BET nitrogen adsorption and desorption method, the catalyst obtained in step (2) has the following pore distribution characteristics:
the pore volume of micropores with a diameter of greater than 0.5nm and less than 2.0nm accounts for 4-28% of the total pore volume, preferably 5-22%, such as 8%, 10%, 12%, 15%, 18% or 20%; the pore volume of mesopores with a diameter of 2 to 50 nm accounts for 6-50% of the total pore volume, preferably 10-40%, such as 15%, 20%, 25%, 30% or 35%.

In a further preferred embodiment, the ratio of the mesopore volume to the micropore volume of the molecular sieve is 0.4-4.5, such as 0.5, 1.0, 1.5, 2.0, 2.5, 3.0, 3.5 or 4.0.

In a further preferred embodiment, the ratio of the mass of the added component calculated as oxide to the product of the mass of the molecular sieve and the ratio of its mesopore volume to the total pore volume is not greater than 0.50, preferably 0.08-0.35, for example, 0.1, 0.12, 0.15, 0.18, 0.20, 0.22, 0.25, 0.28, 0.30 or 0.32.

In a preferred embodiment, the support used in step (1) is selected from alumina, alumina-containing clay, silica, or a combination thereof, preferably selected from alumina, kaolin, attapulgite, bentonite, diatomite, silica or a combination thereof, more preferably alumina.

In a preferred embodiment, the precursor of the added component may be a soluble salt of the added component, preferably a nitrate, a halide, a haloate of the added component or a combination thereof.

In the method of the present application, the shaping in step (1) can adopt conventional shaping methods, such as extrusion shaping, tablet shaping, etc. According to the needs of shaping, a shaping aid, such as nitric acid, an extrusion aid (such as sesbania powder), water, or a combination thereof can be added.

In a preferred embodiment, the drying conditions described in step (1) include: drying temperature being 30-300°C, preferably 60-260°C, drying time being 0.5-72h, preferably 1-48h; calcination conditions are as follows: calcination temperature being 450-700°C, preferably 450-600°C, calcination time being 0.2-30h, preferably 1-24h. The drying and calcining are performed in an oxygen-containing atmosphere (such as air, oxygen).

In a preferred embodiment, among the precursors of the modifying components used in step (2), the precursor of silicon is organic silicon, such as ethyl orthosilicate, silicone oils (preferably selected from phenylmethyl silicone oil, amino silicone oil, hydroxyl silicone oil or a combination thereof), methyl silicate, siloxane monomers with alkyl group of carbon number of 1-4, and halosiloxane with alkyl group of carbon number of 1-4 (such as chlorosilane) monomer or a combination thereof; the precursors of germanium, bismuth, gallium and tin can be their soluble salts, such as nitrates, halides, haloates, sulfates, etc., and the precursors of boron can be boric acid, etc..

In a preferred embodiment, the loading in step (2) is performed in the following manner: the shaped body is first impregnated and loaded by a precursor of silicon, and then is impregnated and loaded by other modifying component selected from germanium, bismuth, tin, gallium and boron or a combination thereof. In a further preferred embodiment, the specific process of step (2) includes: impregnating the shaped body with an impregnation solution containing silicone, drying it to obtain a silicon-containing shaped body; then impregnating the silicon-containing shaped body with an impregnating solution containing a precursor of another modifying component selected from germanium, bismuth, tin, gallium and boron or a combination thereof, and drying and calcining it, to obtain the catalyst. More preferably, the solvent used in the silicone-containing impregnating solution is toluene, an alkane (preferably of carbon number of 5-16) or a combination thereof, and the drying conditions when preparing the silicon-containing shaped body include a drying temperature of 30-200°C, a drying time of 0.1-72 h, the drying is carried out under an inert atmosphere (such as nitrogen). Preferably, the impregnation adopts an equal volume impregnation method.

In a preferred embodiment, the drying conditions described in step (2) include: drying temperature being 30-200°C, preferably 60-150°C, drying time being 0.1-72h, preferably 1-48h; calcination conditions including: calcination temperature being 400-650°C, preferably 450-600°C, and calcination time being 0.5-8h, preferably 1-5h. Preferably, the drying and calcining are performed under an inert atmosphere (such as nitrogen).

In a third aspect, the present application provides an olefin conversion catalyst prepared by the method of the present application.

In a specific embodiment, various characteristics of the olefin conversion catalyst obtained by the method of the present application are as described in the first aspect of the present application, and will not be described again here.

In a fourth aspect, the present application provides an application of the olefin conversion catalyst of the present application in the conversion of olefins in aromatic-rich distillate oil.

In a fifth aspect, the present application provides a method for converting olefins contained in an aromatic-rich distillate oil, including a step of contacting and reacting the aromatic-rich distillate oil containing olefins with the olefin conversion catalyst of the present application.

In a preferred embodiment, the aromatic-rich distillate oil is selected from a reformate, an isomerization reaction product, an extracted aromatic hydrocarbon mixture, or a combination thereof. Further preferably, the distillation range of the aromatic-rich distillate oil is 75-220°C, and the contained aromatic hydrocarbon may be benzene, toluene, C8 aromatic hydrocarbons, C9 aromatic hydrocarbons or a combination thereof. For example, the isomerization reaction product may be a para-xylene-containing product generated by the isomerization reaction of mixed C8 aromatic hydrocarbons after removing para-xylene through adsorption separation in an aromatic hydrocarbons complex unit, which mainly compries ethylbenzene, xylene and a small amount of olefins, which need to be refined to remove the olefins before adsorption separation; the extracted aromatic hydrocarbon mixture can be the liquid extracted from the extraction unit, which mainly contains benzene and toluene, and a small amount of olefins, which needs to be refined to remove olefins to obtain qualified benzene and toluene products.

In a preferred embodiment, the olefin content in the aromatic-rich distillate oil is expressed by bromine index, and the bromine index is 100-2800 mgBr/100g, preferably 300-2000 mgBr/100g.

In a preferred embodiment, the reaction is carried out under non-hydrogen conditions, and the reaction conditions include: reaction temperature being 130-350°C, reaction pressure being 0.5-4.0MPa, and liquid hourly volume space velocity being 0.5-35h^{-1.} Preferably, the reaction conditions include: reaction temperature being 130-260°C, reaction pressure being 0.5-3.0MPa, and liquid hourly volume space velocity being 0.5-8h⁻¹.

### Example

This application will be described in detail below with reference to the examples, but the protection scope of the present application is not limited to the examples.

In the following examples and comparative examples, the pore volume and pore distribution were measured using a liquid nitrogen adsorption and desorption instrument according to the BET method. The measurement method is as follows: test was conducted at the liquid nitrogen saturation temperature (77K), and N₂ isothermal adsorption-desorption curves were obtained through different relative pressures, and pore distribution was obtained by t-plot method. The relative pressure during the experiment was 0.001-0.995. The total pore volume was calculated by the single-point method at a relative pressure of 0.995.

In the following examples and comparative examples, the composition of the catalyst was measured by XRF method, and the instrument was Philips Co Magix 601.

In the following examples and comparative examples, SEM-EDS characterization was completed using a Philips XL30 scanning electron microscope. During measurement, parallel lines were taken on the particles and divided into twenty equal parts in vertical direction for measurement, and the average value was obtained to obtain the results.

### [Example 1]

100g ammonic MCM-22 molecular sieve (SiO₂/Al₂O₃ molar ratio is 15, pore distribution is as follows: pore volume of pore diameter 0.5-2.0 nm accounts for 19% of the total pore volume, pore volume of pore diameter 2-50 nm accounts for 45% of the total pore volume) and 65g pseudo-boehmite (containing 67 wt% alumina) were taken, 12g sesbania powder, 6g of 65% nitric acid, and an aqueous solution of magnesium nitrate were added hereinto, and the mixture was kneaded into shape, then in air atmosphere, was dried at 120°C for 12 hours and calcined at 580°C for 2 hours to obtain a shaped body. Then 100g of the shaped body was taken and immersed in 50g impregnating solution containing phenylmethyl silicone oil (toluene as solvent), then was treated under nitrogen at 70°C for 3 hours to remove the solvent, and dried at 120°C for 10 hours. Then 100g of the sample was taken, and dipped into equal volume of aqueous solution of tin sulfate so that the mass ratio of silica to tin oxide was 2.0, then in a nitrogen atmosphere, was dried at 120°C for 5 hours and calcined at 550°C for 3 hours to obtain a catalyst. In the obtained catalyst, the content of molecular sieve was 64.5%, and the content of support (due to the relative ratio of the molecular sieve to the support used during the shaping is determined, the aluminum content in the molecular sieve can be calculated from the total aluminum content of the obtained catalyst, and then the content of molecular sieve and the content of support of the obtained catalyst are determined, the following is also the same) was 27.5%, the mass content of silica was 3.6%, and the mass content of tin oxide was 1.8% (SEM-EDS characterization shows that its content dispersed on the outer surface of molecular sieve grains is 0.6%), the mass content of magnesium oxide was 2.1% (SEM-EDS characterization shows that its content dispersed on the outer surface of the molecular sieve grains is 2.0%), the ratio of the pore volume of 2-50nm mesopore (accounting for 32% of the total pore volume) to the pore volume of 0.5-2nm micropore (accounting for 11% of the total pore volume) was 2.9. The ratio of the mass of the added component (calculated as oxide) to the product of the mass of the molecular sieve and the ratio of the mesopore volume to the total pore volume of the catalyst was 0.10. The results of BET measurement and t-plot method calculation of the mesopore distribution of the obtained catalyst are shown in Figure 1.

5g of the catalyst was taken, and evaluated by reacting a reformate with a bromine index of 1520mgBr/100g (the olefin content is expressed in terms of bromine index, and the mass content of C8 aromatic hydrocarbons is 52.0%) at 30h⁻¹ and 1.9MPa and at a temperature of 200°C. After 260 hours, the bromine index at outlet exceeded 200 mgBr/100g. The content of C8 aromatic hydrocarbons was evaluated by reacting the above reformate at 2.0h⁻¹, 1.9MPa, and at a temperature of 200°C; and the mass content of C8 aromatic hydrocarbons was 54.3%.

### [Example 2]

100g ammonicc MCM-22 molecular sieve (SiO₂/Al₂O₃ molar ratio is 15, pore distribution is as follows: pore volume of pore diameter 0.5-2.0 nm accounts for 15% of the total pore volume, pore volume of pore diameter 2-50 nm accounts for 35% of the total pore volume) and 65g pseudo-boehmite (containing 67 wt% alumina) were taken, 12g sesbania powder, 6g of 65wt% nitric acid, and an aqueous solution of sodium nitrate were added hereinto, and the mixture was kneaded into shape, then in air atmosphere, was dried at 120°C for 12 hours and calcined at 580°C for 2 hours to obtain a shaped body. Then 100g of the shaped body was taken, and dipped in 50g impregnating solution containing phenylmethyl silicone oil (toluene as solvent), then was treated under nitrogen at 70°C for 3 hours to remove the solvent, and dried at 120°C for 10 hours. Then 100g of the sample was taken, and dipped into equal volume of aqueous solution of bismuth nitrate, so that the mass ratio of silica to bismuth oxide was 4.9, then in a nitrogen atmosphere, was dried at 120°C for 5 hours and calcined at 550°C for 3 hours to obtain a catalyst. In the obtained catalyst, the content of molecular sieve was 62.4%, the content of support was 26.7%, the mass content of silica was 7.8%, the mass content of bismuth oxide was 1.6%, and the mass content of sodium oxide was 1.1%; the ratio of the pore volume of 2-50nm mesopore (accounting for 23.0% of the total pore volume) to the pore volume of 0.5-2nm micropore (accounting for 8.5% of the total pore volume) was 2.7. The ratio of the mass of the added component (calculated as oxide) to the product of the mass of the molecular sieve and the ratio of the mesopore volume to the total pore volume of the catalyst was 0.077.

5g of the catalyst was taken, and evaluated by reacting a reformate with a bromine index of 1520mgBr/100g (the olefin content is expressed in terms of bromine index, and the mass content of C8 aromatic hydrocarbons is 52%) at 30h⁻¹ and 1.9MPa and at a temperature of 200°C. After 240 hours, the bromine index at outlet exceeded 200 mgBr/100g. The content of C8 aromatic hydrocarbons was evaluated by reacting the above reformate at 3.0h^{-1,} 1.9MPa, and at a temperature of 200°C; and the mass content of C8 aromatic hydrocarbons was 55.5%.

### [Example 3]

100g ammonicc USY molecular sieve (SiO₂/Al₂O₃ molar ratio is 12, pore distribution is as follows: pore volume of pore diameter 0.5-2.0 nm accounts for 20% of the total pore volume, pore volume of pore diameter 2-50 nm accounts for 20% of the total pore volume) and 65g pseudo-boehmite (containing 67 wt% alumina) were taken, 12g sesbania powder, 6g of 65wt% nitric acid, and an aqueous solution of magnesium nitrate were added hereinto, and the mixture was kneaded into shape, then in air atmosphere, was dried at 120°C for 12 hours and calcined at 580°C for 2 hours to obtain a shaped body. Then 100g of the shaped body was taken, and dipped in 50g impregnating solution containing ethyl orthosilicate (toluene as solvent), then was treated under nitrogen at 70°C for 3 hours to remove the solvent, and dried at 120°C for 10 hours. Then 100g of the sample was taken, and dipped into equal volume of an ethanol solution of germanium chloride, so that the mass ratio of silica to germanium oxide was 5.0, then in a nitrogen atmosphere, was dried at 120°C for 5 hours and calcined at 550°C for 3 hours in a nitrogen atmosphere to obtain a catalyst. In the obtained catalyst, the content of molecular sieve was 64.3%, the content of support was 27.5%, the mass content of silica was 4.0%, the mass content of germanium oxide was 0.8%, and the mass content of magnesium oxide was 3.0%; the ratio of the pore volume of 2-50nm mesopore (accounting for 13.5% of the total pore volume) to the pore volume of 0.5-2nm micropore (accounting for 11.8% of the total pore volume) was 1.1. The ratio of the mass of the added component (calculated as oxide) to the product of the mass of the molecular sieve and the ratio of the mesopore volume to the total pore volume of the catalyst was 0.346.

5g of the catalyst was taken, and evaluated by reacting a reformate with a bromine index of 1520mgBr/100g (the olefin content is expressed in terms of bromine index, and the mass content of C8 aromatic hydrocarbons is 52%) at 30h⁻¹ and 1.9MPa and at a temperature of 200°C. After 120 hours, the bromine index at outlet exceeded 200 mgBr/100g. The content of C8 aromatic hydrocarbons was evaluated by reacting the above reformate at 2.0h^{-1,} 1.9MPa, and at a temperature of 200°C; and the mass content of C8 aromatic hydrocarbons was 52.5%.

### [Example 4]

100g ammonic MCM-22 molecular sieve (SiO₂/Al₂O₃ molar ratio is 15, pore distribution is as follows: pore volume of pore diameter 0.5-2.0 nm accounts for 25% of the total pore volume, pore volume of pore diameter 2-50 nm accounts for 35% of the total pore volume) and 65g pseudo-boehmite (containing 67 wt% alumina) were taken, 12g sesbania powder, 6g of 65wt% nitric acid, and an aqueous solution of magnesium nitrate were added hereinto, and the mixture was kneaded into shape, then in air atmosphere, was dried at 120°C for 12 hours and calcined at 580°C for 2 hours to obtain a shaped body. Then 100g of the shaped body was taken, and dipped in 50g impregnating solution containing phenylmethyl silicone oil (toluene as solvent), then was treated under nitrogen at 70°C for 3 hours to remove the solvent, and dried at 120°C for 10 hours. Then 100g of the sample was taken, and dipped into equal volume of aqueous solution of boric acid, so that the mass ratio of silica to boron oxide was 2.0, then in a nitrogen atmosphere, was dried at 120°C for 10 hours and calcined at 550°C for 3 hours to obtain a catalyst. In the obtained catalyst, the content of molecular sieve was 65.5%, the content of support was 28.2%, the mass content of silica was 2.0%, the mass content of boron oxide was 1.0%, and the mass content of magnesium oxide was 3%; the ratio of the pore volume of 2-50nm mesopore (accounting for 23.1% of the total pore volume) to the pore volume of 0.5-2nm micropore (accounting for 16% of the total pore volume) was 1.4. The ratio of the mass of the added component (calculated as oxide) to the product of the mass of the molecular sieve and the ratio of the mesopore volume to the total pore volume of the catalyst was 0.20.

5g of the catalyst was taken, and evaluated by reacting a reformate with a bromine index of 910mgBr/100g (the olefin content is expressed in terms of bromine index, and the mass content of C8 aromatic hydrocarbons is 57.1%) at 30h⁻¹ and 1.9MPa and at a temperature of 200°C, and the product added 0.059% of a alkylation product of styrene and xylene (dimethylphenyl,phenyl-1,2-ethane). After 300 hours, the bromine index at outlet exceeded 200 mgBr/100g, and after 300 hours, dimethylphenyl,phenyl-1,2-ethane increased in the product: 0.058%. The content of C8 aromatic hydrocarbons was evaluated by reacting the above reformate at 3.0h^{-1,} 1.9MPa, and at a temperature of 200°C; and the mass content of C8 aromatic hydrocarbons was 57.5%.

### [Example 5]

100g ammonic MCM-22 molecular sieve (SiO₂/Al₂O₃ molar ratio is 15, pore distribution is as follows: pore volume of pore diameter 0.5-2.0 nm accounts for 12% of the total pore volume, pore volume of pore diameter 2-50 nm accounts for 50% of the total pore volume) and 65g pseudo-boehmite (containing 67 wt% alumina) were taken, 12g sesbania powder, 6g of 65wt% nitric acid, and an aqueous solution of magnesium nitrate were added hereinto, and the mixture was kneaded into shape, then in air atmosphere, was dried at 120°C for 12 hours and calcined at 580°C for 2 hours to obtain a shaped body. Then 100g of the shaped body was taken, and dipped in 50g impregnating solution containing phenylmethyl silicone oil (toluene as solvent), then was treated under nitrogen at 70°C for 3 hours to remove the solvent, and dried at 120°C for 10 hours. Then 100g of the sample was taken, and dipped into equal volume of aqueous solution of tin sulfate, so that the mass ratio of silica to tin oxide was 1.0, then in a nitrogen atmosphere, was dried at 120°C for 10 hours and calcined at 650°C for 3 hours to obtain a catalyst. In the obtained catalyst, the content of molecular sieve was 57.2%, the content of support was 24.6%, the mass content of silica was 5.0%, the mass content of tin oxide was 5.0%, and the mass content of magnesium oxide was 7.9%; the ratio of the pore volume of 2-50nm mesopore (accounting for 48% of the total pore volume) to the pore volume of 0.5-2nm micropore (accounting for 9.9% of the total pore volume) was 4.8. The ratio of the mass of the added component (calculated as oxide) to the product of the mass of the molecular sieve and the ratio of the mesopore volume to the total pore volume of the catalyst was 0.288.

5g of the catalyst was taken, and evaluated by reacting a reformate with a bromine index of 910 mgBr/100g (the olefin content is expressed in terms of bromine index, and the mass content of C8 aromatic hydrocarbons is 57.1%) at 30h⁻¹ and 1.9MPa and at a temperature of 200°C. After 500 hours, the bromine index at outlet exceeded 200 mgBr/100g. The content of C8 aromatic hydrocarbons was evaluated by reacting the above reformate at 3.0h^{-1,} 1.9MPa, and at a temperature of 200°C; and the mass content of C8 aromatic hydrocarbons was 58.2%.

### [Example 6]

100g ammonic USY molecular sieve (SiO₂/Al₂O₃ molar ratio is 6, pore distribution is as follows: pore volume of pore diameter 0.5-2.0 nm accounts for 22% of the total pore volume, pore volume of pore diameter 2-50 nm accounts for 33% of the total pore volume) and 65g pseudo-boehmite (containing 67 wt% alumina) were taken, 12g sesbania powder, and an aqueous solution of sodium nitrate and magnesium nitrate were added hereinto, and the mixture was kneaded into shape, then in air atmosphere, was dried at 120°C for 12 hours and calcined at 580°C for 2 hours to obtain a shaped body. Then 100g of the shaped body was taken, and dipped in 50g impregnating solution containing amino silicone oil (heptane as solvent), then was treated under nitrogen at 26°C for 3 hours to remove the solvent, and dried at 120°C for 10 hours. Then 100g of the sample was taken, and dipped into equal volume of aqueous solution of bismuth nitrate, so that the mass ratio of silica to bismuth oxide was 10.0, then in a nitrogen atmosphere, was dried at 120°C for 8 hours and calcined at 650°C for 3 hours to obtain a catalyst. In the obtained catalyst, the content of molecular sieve was 60.7%, the content of support was 26.0%, the mass content of silica was 7.0%, the mass content of bismuth oxide was 0.7%, and the mass content of magnesium oxide was 5.0%, the mass content of sodium oxide was 0.2%; the ratio of the pore volume of 2-50nm mesopore (accounting for 21.3% of the total pore volume) to the pore volume of 0.5-2nm micropore (accounting for 12.8% of the total pore volume) was 1.7. The ratio of the mass of the added component (calculated as oxide) to the product of the mass of the molecular sieve and the ratio of the mesopore volume to the total pore volume of the catalyst was 0.402.

5g of the catalyst was taken, and evaluated by reacting a reformate with a bromine index of 1520mgBr/100g (the olefin content is expressed in terms of bromine index, and the mass content of C8 aromatic hydrocarbons is 57.1%) at 10h⁻¹ and 1.9MPa and at a temperature of 180°C. After 160 hours, the bromine index at outlet exceeded 200 mgBr/100g. The content of C8 aromatic hydrocarbons was evaluated by reacting the above reformate at 2.0h⁻¹ and 1.9MPa, and at a temperature of 180°C; and the mass content of C8 aromatic hydrocarbons was 57.5%.

### [Example 7]

100g ammonic USY molecular sieve (SiO₂/Al₂O₃ molar ratio is 6, pore distribution is as follows: pore volume of pore diameter 0.5-2.0 nm accounts for 20% of the total pore volume, pore volume of pore diameter 2-50 nm accounts for 33% of the total pore volume) and 65g pseudo-boehmite (containing 67 wt% alumina) were taken, 12g sesbania powder, and an aqueous solution of sodium nitrate and magnesium nitrate were added hereinto, and the mixture was kneaded into shape, then in air atmosphere, was dried at 120°C for 12 hours and calcined at 580°C for 2 hours to obtain a shaped body. Then 100g of the sample was taken, and dipped in 50g impregnating solution containing amino silicone oil (heptane as solvent), then was treated under nitrogen at 26°C for 3 hours to remove the solvent, and dried at 120°C for 10 hours. Then 100g of the sample was taken, and dipped into equal volume of aqueous solution of tin sulfate, so that the mass ratio of silica to tin oxide was 10.0, then in a nitrogen atmosphere, was dried at 120°C for 8 hours and calcined at 500°C for 3 hours to obtain a catalyst. In the obtained catalyst, the content of molecular sieve was 66.0%, the content of support was 28.1%, the mass content of silica was 3.5%, the mass content of tin oxide was 0.35%, and the mass content of magnesium oxide was 0.8%, the mass content of sodium oxide was 0.6%; the ratio of the pore volume of 2-50nm mesopore (accounting for 22.6% of the total pore volume) to the pore volume of 0.5-2nm micropore (accounting for 13.0% of the total pore volume) was 1.7. The ratio of the mass of the added component (calculated as oxide) to the product of the mass of the molecular sieve and the ratio of the mesopore volume to the total pore volume of the catalyst was 0.094.

5g of the catalyst was taken, and evaluated by reacting a reformate with a bromine index of 1520 mgBr/100g (the olefin content is expressed in terms of bromine index, and the mass content of C8 aromatic hydrocarbons is 52.0%) at 8h⁻¹ and 1.9MPa and at a temperature of 170°C. After 90 hours, the bromine index at outlet exceeded 200 mgBr/100g. The content of C8 aromatic hydrocarbons was evaluated by reacting the above reformate at 2.0h^{-1,} 1.9MPa, and at a temperature of 170°C; and the mass content of C8 aromatic hydrocarbons was 53.1%.

### [Example 8]

100g ammonic USY molecular sieve (SiO₂/Al₂O₃ molar ratio is 6, pore distribution is as follows: pore volume of pore diameter 0.5-2.0 nm accounts for 21% of the total pore volume, pore volume of pore diameter 2-50 nm accounts for 30% of the total pore volume) and 30g silica sol (containing 40wt% silicon oxide) and 20g kaolin were taken, 12g sesbania powder, and an aqueous solution of sodium nitrate and calcium nitrate were added hereinto, and the mixture was kneaded into shape, then in air atmosphere, was dried at 120°C for 12 hours and calcined at 580°C for 2 hours to obtain a shaped body. Then 100g of the shaped body was taken, and dipped in 50g impregnating solution containing methyl silicone oil (heptane as solvent), then was treated under nitrogen at 26°C for 3 hours to remove the solvent, and dried at 120°C for 10 hours. Then 100g of the sample was taken, and dipped into equal volume of aqueous solution of bismuth nitrate, so that the mass ratio of silica to bismuth oxide was 10.0, then in a nitrogen atmosphere, was dried at 120°C for 8 hours and calcined at 550°C for 3 hours to obtain a catalyst. In the obtained catalyst, the content of molecular sieve was 64.3%, the content of support was 27.7%, the mass content of silica added component was 6.0%, the mass content of bismuth oxide was 0.6%, and the mass content of calcium oxide was 0.3%, the mass content of sodium oxide was 1.2%; the ratio of the pore volume of 2-50nm mesopore (accounting for 23.1% of the total pore volume) to the pore volume of 0.5-2nm micropore (accounting for 13.1% of the total pore volume) was 1.8. The ratio of the mass of the added component (calculated as oxide) to the product of the mass of the molecular sieve and the ratio of the mesopore volume to the total pore volume of the catalyst was 0.093.

5g of the catalyst was taken, and evaluated by reacting a reformate with a bromine index of 1106mgBr/100g (the olefin content is expressed in terms of bromine index, and the mass content of C8 aromatic hydrocarbons is 48.1%) at 30h⁻¹ and 1.9MPa and at a temperature of 280°C. After 260 hours, the bromine index at outlet exceeded 200 mgBr/100g. The content of C8 aromatic hydrocarbons was evaluated by reacting the above reformate at 2.0h^{-1,} 1.9MPa, and at a temperature of 200°C; and the mass content of C8 aromatic hydrocarbons was 50.1%.

### [Example 9]

90g ammonic USY molecular sieve (SiO₂/Al₂O₃ molar ratio is 6, pore distribution is as follows: pore volume of pore diameter 0.5-2.0 nm accounts for 11% of the total pore volume, pore volume of pore diameter 2-50 nm accounts for 29% of the total pore volume), 30g diatomite and 30g attapulgite were taken, 12g sesbania powder, and an aqueous solution of magnesium nitrate were added hereinto, and the mixture was kneaded into shape, then in air atmosphere, was dried at 120°C for 12 hours and calcined at 580°C for 2 hours to obtain a shaped body. Then 100g of the shaped body was taken, and dipped in 50g impregnating solution containing chlorosilane (heptane as solvent), then was treated under nitrogen at 26°C for 3 hours to remove the solvent, and dried at 120°C for 10 hours. Then 100g of the sample was taken, and dipped into equal volume of aqueous solution of bismuth nitrate, so that the mass ratio of silica to bismuth oxide was 5.0, then in a nitrogen atmosphere, was dried at 120°C for 8 hours and calcined at 550°C for 3 hours to obtain a catalyst. In the obtained catalyst, the content of molecular sieve was 55.2%, the content of support was 36.6%, the mass content of silica was 5.0%, the mass content of bismuth oxide was 1.0%, and the mass content of magnesium oxide was 2.0%; the ratio of the pore volume of 2-50nm mesopore (accounting for 17.0% of the total pore volume) to the pore volume of 0.5-2nm micropore (accounting for 5.6% of the total pore volume) was 3.0. The ratio of the mass of the added component (calculated as oxide) to the product of the mass of the molecular sieve and the ratio of the mesopore volume to the total pore volume of the catalyst was 0.213.

5g of the catalyst was taken, and evaluated by reacting a reformate with a bromine index of 1106mgBr/100g (the olefin content is expressed in terms of bromine index, and the mass content of C8 aromatic hydrocarbons is 48.1%) at 6h⁻¹ and 1.9MPa and at a temperature of 160°C. After 150 hours, the bromine index at outlet exceeded 200 mgBr/100g. The content of C8 aromatic hydrocarbons was evaluated by reacting the above reformate at 2.0h^{-1,} 1.9MPa, and at a temperature of 200°C; and the mass content of C8 aromatic hydrocarbons was 48.6%.

### [Example 10]

100g ammonic β zeolite(SiO₂/Al₂O₃ molar ratio is 19, pore distribution is as follows: pore volume of pore diameter 0.5-2.0 nm accounts for 35% of the total pore volume, pore volume of pore diameter 2-50 nm accounts for 30% of the total pore volume), 40g silica sol (containing 40wt% silicon oxide) were taken, 12g sesbania powder, and an aqueous solution of magnesium nitrate were added hereinto, and the mixture was kneaded into shape, then in air atmosphere, was dried at 120°C for 12 hours and calcined at 550°C for 2 hours to obtain a shaped body. Then 100g of the shaped body was taken, and dipped in 50g impregnating solution containing ethyl orthosilicate (ethanol as solvent), then was treated under nitrogen at 26°C for 3 hours to remove the solvent, and dried at 120°C for 10 hours. Then 100g of the sample was taken, and dipped into equal volume of aqueous solution of tin sulfate, so that the mass ratio of silica to tin oxide was 1.0, then in a nitrogen atmosphere, was dried at 120°C for 8 hours and calcined at 500°C for 3 hours to obtain a catalyst. In the obtained catalyst, the content of molecular sieve was 81.8%, the content of support was 13.0%, the mass content of silica was 2.0%, the mass content of tin oxide was 2.0%, and the mass content of magnesium oxide was 1.0%; the ratio of the pore volume of 2-50nm mesopore (accounting for 25.6% of the total pore volume) to the pore volume of 0.5-2nm micropore (accounting for 27.1% of the total pore volume) was 0.94. The ratio of the mass of the added component (calculated as oxide) to the product of the mass of the molecular sieve and the ratio of the mesopore volume to the total pore volume of the catalyst was 0.048.

5g of the catalyst was taken, and evaluated by reacting a reformate with a bromine index of 1106mgBr/100g (the olefin content is expressed in terms of bromine index, and the mass content of C8 aromatic hydrocarbons is 48.1%) at 15h⁻¹ and 1.9MPa and at a temperature of 195°C. After 210 hours, the bromine index at outlet exceeded 200 mgBr/100g. The content of C8 aromatic hydrocarbons was evaluated by reacting the above reformate at 2.0h^{-1,} 1.9MPa, and at a temperature of 200°C; and the mass content of C8 aromatic hydrocarbons was 49.8%.

### [Example 11]

100g ammonic MCM-56 molecular sieve (SiO₂/Al₂O₃ molar ratio is 20, pore distribution is as follows: pore volume of pore diameter 0.5-2.0 nm accounts for 20% of the total pore volume, pore volume of pore diameter 2-50 nm accounts for 48% of the total pore volume), 40g silica sol (containing 40wt% silicon oxide) were taken, 12g sesbania powder, and an aqueous solution of magnesium nitrate were added hereinto, and the mixture was kneaded into shape, then in air atmosphere, was dried at 120°C for 12 hours and calcined at 550°C for 2 hours to obtain a shaped body. Then 100g of the shaped body was taken, and dipped in 50g impregnating solution containing ethyl orthosilicate (ethanol as solvent), then was treated under nitrogen at 26°C for 3 hours to remove the solvent, and dried at 120°C for 10 hours. Then 100g of the sample was taken, and dipped into equal volume of aqueous solution of tin sulfate, then in a nitrogen atmosphere, was dried at 120°C for 8 hours and calcined at 550°C for 3 hours to obtain a catalyst. In the obtained catalyst, the content of molecular sieve was 73.5%, the content of support was 11.7%, the mass content of silica was 5.2%, the mass content of tin oxide was 1.8%, and the mass content of magnesium oxide was 7.5%; the ratio of the pore volume of 2-50nm mesopore (accounting for 38.1% of the total pore volume) to the pore volume of 0.5-2nm micropore (accounting for 13.5% of the total pore volume) was 2.82. The ratio of the mass of the added component (calculated as oxide) to the product of the mass of the molecular sieve and the ratio of the mesopore volume to the total pore volume of the catalyst was 0.268.

5g of the catalyst was taken, and evaluated by reacting reformate with a bromine index of 1106mgBr/100g (the olefin content is expressed in terms of bromine index, and the mass content of C8 aromatic hydrocarbons is 48.1%) at 30h⁻¹ and 1.9MPa and at a temperature of 200°C. After 700 hours, the bromine index at outlet exceeded 200 mgBr/100g. The content of C8 aromatic hydrocarbons was evaluated by reacting the above reformate at 3.0h^{-1,} 1.9MPa, and at a temperature of 200°C; and the mass content of C8 aromatic hydrocarbons was 50.1%.

5g of the catalyst was taken, and evaluated by using a reaction of isomerization products with a bromine index of 208mgBr/100g (the olefin content is expressed in terms of bromine index, and the mass content of C8 aromatic hydrocarbons is 98.5%, the mass content of non-aromatic hydrocarbons is 1.41%, and the mass content of C9 aromatic hydrocarbons is 0.09%.) at 30h⁻¹ and 1.9MPa and at a temperature of 175°C. After 650 hours, the bromine index at outlet exceeded 20 mgBr/100g. The content of C8 aromatic hydrocarbons was evaluated by reacting the above reformate at 5.0h⁻¹, 1.9MPa, and at a temperature of 170°C; and the mass content of C8 aromatic hydrocarbons was 98.49%.

5g of the catalyst was taken, and evaluated by using a reaction of isomerization products with a bromine index of 180mgBr/100g (the olefin content is expressed in terms of bromine index, the mass content of benzene is 32.02%, the mass content of non-aromatic hydrocarbons is 0.05%, and the mass content of toluene aromatic hydrocarbons is 67.93%) at 36h⁻¹ and 1.9MPa and at a temperature of 180°C. After 750 hours, the bromine index at outlet exceeded 20 mgBr/100g. The content of aromatic hydrocarbons was evaluated by reacting the above raw materials at 5.0h⁻¹, 1.9MPa, and at a temperature of 170°C; and the mass content of benzene and toluene was 99.95%.

### [Example 12]

100g ammonic USY molecular sieve (SiO₂/Al₂O₃ molar ratio is 12, pore distribution is as follows: pore volume of pore diameter 0.5-2.0 nm accounts for 20% of the total pore volume, pore volume of pore diameter 2-50 nm accounts for 20.5% of the total pore volume), 65g pseudo-boehmite (contains 67wt% alumina) were taken, 12g sesbania powder, 6g of 65wt% nitric acid, and an aqueous solution of magnesium nitrate were added hereinto, and the mixture was kneaded into shape, then in air atmosphere, was dried at 120°C for 12 hours and calcined at 580°C for 2 hours to obtain a shaped body. Then 100g sample was taken, and dipped in equal volume of ethanol solution of germanium chloride, then was dried under nitrogen at 120°C for 5 hours, and calcined under nitrogen at 550°C for 3 hours to obtain a catalyst. In the obtained catalyst, the content of molecular sieve was 67.1%, the content of support was 28.9%, the mass content of germanium oxide was 0.8%, and the mass content of magnesium oxide was 3.0%; the ratio of the pore volume of 2-50nm mesopore (accounting for 13.0% of the total pore volume) to the pore volume of 0.5-2nm micropore (accounting for 12.9% of the total pore volume) was 1.01. The ratio of the mass of the added component (calculated as oxide) to the product of the mass of the molecular sieve and the ratio of the mesopore volume to the total pore volume of the catalyst was 0.344.

5g of the catalyst was taken, and evaluated by reacting a reformate with a bromine index of 1520mgBr/100g (the olefin content is expressed in terms of bromine index, and the mass content of C8 aromatic hydrocarbons is 52.0%) at 10h⁻¹ and 1.9MPa and at a temperature of 200°C. After 180 hours, the bromine index at outlet exceeded 200 mgBr/100g. The content of C8 aromatic hydrocarbons was evaluated by reacting the above reformate at 1.5h⁻¹, 1.9MPa, and at a temperature of 200°C; and the mass content of C8 aromatic hydrocarbons was 52.6%.

### [Example 13]

100g ammonic MCM-22 molecular sieve (SiO₂/Al₂O₃ molar ratio is 15, pore distribution is as follows: pore volume of pore diameter 0.5-2.0 nm accounts for 25% of the total pore volume, pore volume of pore diameter 2-50 nm accounts for 35% of the total pore volume) and 65g pseudo-boehmite (containing 67 wt% alumina) were taken, 12g sesbania powder, 6g of 65wt% nitric acid, and an aqueous solution of calcium nitrate were added hereinto, and the mixture was kneaded into shape, then in air atmosphere, was dried at 120°C for 12 hours and calcined at 580°C for 2 hours to obtain a shaped body. Then 100g of the shaped body was taken, and dipped in 50g impregnating solution containing phenylmethyl silicone oil (toluene as solvent), then was treated under nitrogen at 70°C for 3 hours to remove the solvent, and dried at 120°C for 10 hours. Then 100g of the sample was taken, and dipped into equal volume of aqueous solution of boric acid, so that the mass ratio of silica to boron oxide was 2.0, then in a nitrogen atmosphere, was dried at 120°C for 10 hours and calcined at 550°C for 3 hours to obtain a catalyst. In the obtained catalyst, the content of molecular sieve was 64.5%, the content of support was 27.8%, the mass content of silica was 2.2%, the mass content of calcium oxide was 1.2%, and the mass content of boron oxide was 4.1%; the ratio of the pore volume of 2-50nm mesopore (accounting for 23.6% of the total pore volume) to the pore volume of 0.5-2nm micropore (accounting for 15.2% of the total pore volume) was 1.57. The ratio of the mass of the added component (calculated as oxide) to the product of the mass of the molecular sieve and the ratio of the mesopore volume to the total pore volume of the catalyst was 0.267.

5g of the catalyst was taken, and evaluated by reacting a reformate with a bromine index of 910 mgBr/100g (the olefin content is expressed in terms of bromine index, and the mass content of C8 aromatic hydrocarbons is 57.1%) at 30h⁻¹ and 1.9MPa and at a temperature of 200°C. After 358 hours, the bromine index at outlet exceeded 200 mgBr/100g, and after 300 hours, dimethylphenyl,phenyl-1,2-ethane increased in the product: 0.058%. The content of C8 aromatic hydrocarbons was evaluated by reacting the above reformate at 3.0h^{-1,} 1.9MPa, and at a temperature of 200°C; and the mass content of C8 aromatic hydrocarbons was 57.4%.

### [Example 14]

100g ammonic SAPO-5 molecular sieve (SiO₂/Al₂O₃ molar ratio is 0.4, SiO₂/P₂O₅ molar ratio is 1, pore distribution is as follows: pore volume of pore diameter 0.5-2.0 nm accounts for 31.0% of the total pore volume, pore volume of pore diameter 2-50 nm accounts for 15.1% of the total pore volume) and 98.2g pseudo-boehmite (containing 67 wt% alumina) were taken, 12g sesbania powder, 6g of 65wt% nitric acid, and a solution of 2.7g sodium nitrate dissolved in 10g water were added hereinto, and the mixture was kneaded into shape, then in air atmosphere, was dried at 120°C for 12 hours and calcined at 580°C for 2 hours to obtain a shaped body. Then 100g of the shaped body was taken, and dipped in 50g impregnating solution containing methyl silicone oil (toluene as solvent, silicone oil viscosity being 100cps), then was treated under nitrogen at 70°C for 3 hours to remove the solvent, and dried at 150°C for 10 hours. Then 100g of the sample was taken, and dipped into equal volume of aqueous solution of tin sulfate, so that the mass ratio of silica to tin oxide was 1.0, then in a nitrogen atmosphere, was dried at 120°C for 10 hours and calcined at 550°C for 3 hours to obtain a catalyst. In the obtained catalyst, the content of molecular sieve was 59.0%, the content of support was 38.8%, the mass content of silica was 2.2%, the mass content of sodium oxide was 0.6%, and the mass content of tin oxide was 0.5%; the ratio of the pore volume of 2-50nm mesopore (accounting for 9.1% of the total pore volume) to the pore volume of 0.5-2nm micropore (accounting for 17.7% of the total pore volume) was 0.51. The ratio of the mass of the added component (calculated as oxide) to the product of the mass of the molecular sieve and the ratio of the mesopore volume to the total pore volume of the catalyst was 0.11.

5g of the catalyst was taken, and evaluated by reacting a reformate with a bromine index of 630 mgBr/100g (the olefin content is expressed in terms of bromine index, and the mass content of C8 aromatic hydrocarbons is 52.8%) at 10h⁻¹ and 1.9MPa and at a temperature of 220°C. After 170 hours, the bromine index at outlet exceeded 200 mgBr/100g. The content of C8 aromatic hydrocarbons was evaluated by reacting the above reformate at 2.0h^{-1,} 1.9MPa, and at a temperature of 200°C; and the mass content of C8 aromatic hydrocarbons was 53.5%.

### [Example 15]

20g ammonic SAPO-37 molecular sieve (SiO₂/Al₂O₃ molar ratio is 0.43, SiO₂/P₂O₅ molar ratio is 1, pore distribution is as follows: pore volume of pore diameter 0.5-2.0 nm accounts for 31.1% of the total pore volume, pore volume of pore diameter 2-50 nm accounts for 14.9% of the total pore volume) and 27.0g pseudo-boehmite (containing 67 wt% alumina) were taken, 4g sesbania powder, 1.5g of 65wt% nitric acid, and solution of 0.7g potassium nitrate dissolved in 4g water were added hereinto, and the mixture was kneaded into shape, then in air atmosphere, was dried at 120°C for 12 hours and calcined at 580°C for 2 hours to obtain a shaped body. Then 20g of the shaped body was taken, and dipped in 10g impregnating solution containing methyl silicone oil (toluene as solvent, 10% silicone oil, silicone oil viscosity being 100cps), then was treated under nitrogen at 70°C for 3 hours to remove the solvent, and dried at 150°C for 10 hours. Then 15g of the sample was taken, and dipped into equal volume of aqueous solution of tin sulfate, so that the mass ratio of silica to tin oxide was 1.0, then in a nitrogen atmosphere, was dried at 120°C for 10 hours and calcined at 550°C for 3 hours to obtain a catalyst. In the obtained catalyst, the content of molecular sieve was 53.8%, the content of support was 43.8%, the mass content of silica was 0.8%, the mass content of potassium oxide was 0.6%, and the mass content of tin oxide was 0.5%; the ratio of the pore volume of 2-50nm mesopore (accounting for 8.1% of the total pore volume) to the pore volume of 0.5-2nm micropore (accounting for 16.2% of the total pore volume) was 0.50. The ratio of the mass of the added component (calculated as oxide) to the product of the mass of the molecular sieve and the ratio of the mesopore volume to the total pore volume of the catalyst was 0.16.

5g of the catalyst was taken, and evaluated by reacting a reformate with a bromine index of 630 mgBr/100g (the olefin content is expressed in terms of bromine index, and the mass content of C8 aromatic hydrocarbons is 52.8%) at 8h⁻¹ and 2.9MPa and at a temperature of 300°C. After 190 hours, the bromine index at outlet exceeded 200 mgBr/100g. The content of C8 aromatic hydrocarbons was evaluated by reacting the above reformate at 2.0h^{-1,} 1.9MPa, and at a temperature of 200°C; and the mass content of C8 aromatic hydrocarbons was 53.0%.

### [Comparative Example 1]

100g ammonic USY molecular sieve (SiO₂/Al₂O₃ molar ratio is 12, pore distribution is as follows: pore volume of pore diameter 0.5-2.0 nm accounts for 20% of the total pore volume, pore volume of pore diameter 2-50 nm accounts for 20.5% of the total pore volume), 65g pseudo-boehmite (containing 67wt% alumina) were taken, 12g sesbania powder, 6g of 65wt% nitric acid and an appropriate amount of water were added hereinto, and the mixture was kneaded into shape, then in air atmosphere, was dried at 120°C for 10 hours and calcined at 550°C for 3 hours to obtain a catalyst. The obtained catalyst did not contain the added component and the modifying component, had a molecular sieve content of 69.7%, a support content of 30.2%, the ratio of a mesopore volume of 2-50nm (accounting for 14.0% of the total pore volume) to a micropore volume of 0.5-2nm ( accounting for 13.8% of the total pore volume) was 1.01.

5g of the catalyst was taken, and evaluated by reacting a reformate with a bromine index of 1520mgBr/100g (the olefin content is expressed in terms of bromine index, and the mass content of C8 aromatic hydrocarbons is 52.5%) at 30h⁻¹ and 1.9MPa and at a temperature of 200°C. After 30 hours, the bromine index at outlet exceeded 200 mgBr/100g. The content of C8 aromatic hydrocarbons was evaluated by reacting the above reformate at 2.0h^{-1,} 1.9MPa, and at a temperature of 200°C; and the mass content of C8 aromatic hydrocarbons was 51.2%.

### [Comparative Example 2]

100g ammonic USY molecular sieve (SiO₂/Al₂O₃ molar ratio is 12, pore distribution is as follows: pore volume of pore diameter 0.5-2.0 nm accounts for 20% of the total pore volume, pore volume of pore diameter 2-50 nm accounts for 20.5% of the total pore volume), 65g pseudo-boehmite (containing 67wt% alumina) were taken, 12g sesbania powder, 6g of 65wt% nitric acid were added hereinto, and the mixture was kneaded into shape, then in air atmosphere, was dried at 120°C for 12 hours and calcined at 580°C for 2 hours to obtain a shaped body. Then 100g of the shaped body was taken, and dipped in 50g impregnating solution containing ethyl orthosilicate (toluene as solvent), then was treated under nitrogen at 70°C for 3 hours to remove the solvent, and dried at 120°C for 10 hours. Then 100g of the sample was taken, and dipped into equal volume of ethanol solution of germanium chloride, so that the mass ratio of silica to germanium oxide was 5.0, then, was dried in nitrogen atmosphere at 120°C for 5 hours and calcined in nitrogen atmosphere at 550°C for 3 hours to obtain a catalyst. The obtained catalyst did not contain the added components, had a molecular sieve content of 66.4%, a support content of 28.5%, a mass content of silica of 4.0%, and a mass content of germanium oxide of 0.8%; the ratio of a mesopore volume of 2-50nm (accounting for 13.4% of the total pore volume) to a micropore volume of 0.5-2nm (accounting for 13% of the total pore volume) was 1.03.

5g of the catalyst was taken, and evaluated by reacting a reformate with a bromine index of 1520mgBr/100g (the olefin content was expressed in terms of bromine index, and the mass content of C8 aromatic hydrocarbons was 52.0%) at 30h⁻¹ and 1.9MPa and at a temperature of 200°C. After 82 hours, the bromine index at outlet exceeded 200 mgBr/100g. The content of C8 aromatic hydrocarbons was evaluated by reacting the above reformate at 2.0h^{-1,} 1.9MPa, and at a temperature of 200°C; and the mass content of C8 aromatic hydrocarbons was 51.5%.

### [Comparative Example 3]

100g ammonic MCM-22 molecular sieve (SiO₂/Al₂O₃ molar ratio is 15, pore distribution is as follows: pore volume of pore diameter 0.5-2.0 nm accounts for 12% of the total pore volume, pore volume of pore diameter 2-50 nm accounts for 20% of the total pore volume), 65g pseudo-boehmite (containing 67wt% alumina) were taken, 12g sesbania powder, 6g of 65wt% nitric acid, an aqueous solution of magnesium nitrate were added hereinto, and the mixture was kneaded into shape, then in air atmosphere, was dried at 120°C for 12 hours and calcined at 580°C for 2 hours to obtain a shaped body. Then 100g of the shaped body was taken, and dipped in 50g impregnating solution containing phenyl methyl silicone oil (toluene as solvent), then was treated under nitrogen at 70°C for 3 hours to remove the solvent, and dried at 120°C for 10 hours. Then 100g of the sample was taken, and dipped into equal volume of aqueous solution of tin sulfate, so that the mass ratio of silica to tin oxide was 1.0, then in a nitrogen atmosphere, was dried at 120°C for 10 hours and calcined in a nitrogen atmosphere at 650°C for 3 hours to obtain a catalyst. In the obtained catalyst, the content of molecular sieve was 47.3%, the content of support was 20.3%, the mass content of silica was 5.0%, and the mass content of tin oxide was 15.1%, the mass content of magnesium oxide was 12.1%; the ratio of a mesopore volume of 2-50nm (accounting for 12.3% of the total pore volume) to a micropore volume of 0.5-2nm (accounting for 6.7% of the total pore volume) was 1.84; the ratio of the mass of the added components (calculated as oxides) to the product of the mass of the molecular sieve and the ratio of the mesopore volume to the total pore volume of the catalyst was 2.1.

5g of the catalyst was taken, and evaluated by reacting a reformate with a bromine index of 910mgBr/100g (the olefin content is expressed in terms of bromine index, and the mass content of C8 aromatic hydrocarbons is 57.1%) at 30h⁻¹ and 1.9MPa and at a temperature of 200°C. After 228 hours, the bromine index at outlet exceeded 200 mgBr/100g. The content of C8 aromatic hydrocarbons was evaluated by reacting the above reformate at 3.0h⁻¹, 1.9MPa, and at a temperature of 200°C; and the mass content of C8 aromatic hydrocarbons was 56.8%.

### [Comparative Example 4]

100g ammonic MCM-22 molecular sieve (SiO₂/Al₂O₃ molar ratio is 18, pore distribution is as follows: pore volume of pore diameter 0.5-2.0 nm accounts for 5% of the total pore volume, pore volume of pore diameter 2-50 nm accounts for 36% of the total pore volume), 65g pseudo-boehmite (containing 67wt% alumina) were taken, 12g sesbania powder, 6g of 65wt% nitric acid, an aqueous solution of bismuth nitrate were added hereinto, and the mixture was kneaded into shape, then in air atmosphere, was dried at 120°C for 12 hours and calcined at 580°C for 2 hours to obtain a shaped body. Then 100g of the shaped body was taken, and dipped in 50g impregnating solution containing phenyl methyl silicone oil (toluene as solvent), then was treated under nitrogen at 70°C for 3 hours to remove the solvent, and dried at 120°C for 10 hours. Then 100g of the sample was taken, and dipped into equal volume of aqueous solution of boric acid, so that the mass ratio of silica to boron oxide was 2.0, then in a nitrogen, was dried atmosphere at 120°C for 10 hours and calcined in a nitrogen atmosphere at 550°C for 3 hours to obtain a catalyst. In the obtained catalyst, the content of molecular sieve was 64.5%, the content of support was 27.8%, the mass content of silica was 2.2%, the mass content of bismuth oxide was 1.2%, and the mass content of boron oxide was 4.1%; the ratio of a mesopore volume of 2-50nm (accounting for 22.2% of the total pore volume) to a micropore volume of 0.5-2nm (accounting for 3.0% of the total pore volume) was 7.40; the ratio of the mass of the added components (calculated as oxides) to the product of the mass of the molecular sieve and the ratio of the mesopore volume to the total pore volume of the catalyst was 0.286.

5g of the catalyst was taken, and evaluated by reacting a reformate with a bromine index of 9100mgBr/100g (the olefin content is expressed in terms of bromine index, and the mass content of C8 aromatic hydrocarbons is 57.1%) at 30h⁻¹ and 1.9MPa and at a temperature of 200°C. After 358 hours, the bromine index at outlet exceeded 200 mgBr/100g, and after 300 hours, dimethylphenyl,phenyl-1,2-ethane increased in the product: 0.058%. The content of C8 aromatic hydrocarbons was evaluated by reacting the above reformate at 3.0h⁻¹, 1.9MPa, and at a temperature of 200°C; and the mass content of C8 aromatic hydrocarbons was 57.0%.

### [Comparative Example 5]

100g ammonic MCM-22 molecular sieve (SiO₂/Al₂O₃ molar ratio is 20, pore distribution is as follows: pore volume of pore diameter 0.5-2.0 nm accounts for 25% of the total pore volume, pore volume of pore diameter 2-50 nm accounts for 8% of the total pore volume), 65g pseudo-boehmite (containing 67wt% alumina) were taken, 12g sesbania powder, 6g of 65wt% nitric acid, and an aqueous solution of bismuth nitrate were added hereinto, and the mixture was kneaded into shape, then in air atmosphere, was dried at 120°C for 12 hours and calcined at 580°C for 2 hours to obtain a shaped body. Then 100g of the shaped body was taken, and dipped in 50g impregnating solution containing phenyl methyl silicone oil (toluene as solvent), then was treated under nitrogen at 70°C for 3 hours to remove the solvent, and dried at 120°C for 10 hours. Then 100g of the sample was taken, and dipped into equal volume of aqueous solution of boric acid, so that the mass ratio of silica to boron oxide was 2.0, then in a nitrogen, was dried atmosphere at 120°C for 10 hours and calcined in a nitrogen atmosphere at 550°C for 3 hours to obtain a catalyst. In the obtained catalyst, the content of molecular sieve was 64.5%, the content of support was 27.8%, the mass content of silica was 2.2%, the mass content of bismuth oxide was 1.2%, and the mass content of boron oxide was 4.1%; the ratio of a mesopore volume of 2-50nm (accounting for 6.5% of the total pore volume) to a micropore volume of 0.5-2nm (accounting for 15.9% of the total pore volume) was 0.41; the ratio of the mass of the added components (calculated as oxides) to the product of the mass of the molecular sieve and the ratio of the mesopore volume to the total pore volume of the catalyst was 0.954.

5g of the catalyst was taken, and evaluated by reacting a reformate with a bromine index of 910 mgBr/100g (the olefin content is expressed in terms of bromine index, and the mass content of C8 aromatic hydrocarbons is 57.1%) at 30h⁻¹ and 1.9MPa and at a temperature of 200°C. After 358 hours, the bromine index at outlet exceeded 200 mgBr/100g, and after 300 hours, dimethylphenyl,phenyl-1,2-ethane increased in the product: 0.058%. The content of C8 aromatic hydrocarbons was evaluated by reacting the above reformate at 3.0h⁻¹, 1.9MPa, and at a temperature of 200°C; and the mass content of C8 aromatic hydrocarbons was 56.9%.

### [Comparative Example 6]

100g ammonic MCM-22 molecular sieve (SiO₂/Al₂O₃ molar ratio is 20, pore distribution is as follows: pore volume of pore diameter 0.5-2.0 nm accounts for 25% of the total pore volume, pore volume of pore diameter 2-50 nm accounts for 8% of the total pore volume), 65g pseudo-boehmite (containing 67wt% alumina) were taken, 12g sesbania powder, 6g of 65wt% nitric acid were added hereinto, and the mixture was kneaded into shape, then in air atmosphere, was dried at 120°C for 12 hours and calcined at 580°C for 2 hours to obtain a shaped body. Then 100g of the shaped catalyst was taken and dipped in an equal volume of 40g of magnesium nitrate-containing solution, kept at room temperature for 7 hours, and dried at 180°C for 6 hours; then dipped in 50g impregnating solution containing phenyl methyl silicone oil (toluene as solvent), then was treated under nitrogen at 70°C for 3 hours to remove the solvent, and dried at 120°C for 10 hours. Then 100g of the sample was taken, and dipped into equal volume of aqueous solution of boric acid, so that the mass ratio of silica to boron oxide was 2.0, then in a nitrogen, was dried atmosphere at 120°C for 10 hours and calcined at 550°C for 3 hours to obtain a catalyst. In the obtained catalyst, the content of molecular sieve was 64.0%, the content of support was 27.9%, the mass content of magnesium oxide was 1.5%, the mass content of silica was 2.2%, and the mass content of boron oxide was 4.1%; the ratio of a mesopore volume of 2-50nm (accounting for 7.2% of the total pore volume) to a micropore volume of 0.5-2nm (accounting for 15.4% of the total pore volume) was 0.47; the ratio of the mass of the added components (calculated as oxides) to the product of the mass of the molecular sieve and the ratio of the mesopore volume to the total pore volume of the catalyst was 0.416.

5g of the catalyst was taken, and evaluated by reacting a reformate with a bromine index of 910 mgBr/100g (the olefin content is expressed in terms of bromine index, and the mass content of C8 aromatic hydrocarbons is 57.1%) at 30h⁻¹ and 1.9MPa and at a temperature of 200°C. After 158 hours, the bromine index at outlet exceeded 200 mgBr/100g. The content of C8 aromatic hydrocarbons was evaluated by reacting the above reformate at 3.0h⁻¹, 1.9MPa, and at a temperature of 200°C; and the mass content of C8 aromatic hydrocarbons was 56.4%.

The preferred embodiments of the present application have been described in detail above. However, the present application was not limited to the specific details in the above-mentioned embodiments. Within the scope of the technical concept of the present application, a variety of simple modifications can be made to the technical solutions of the present application, and these simple modifications all fall within the protection scope of the present application.

In addition, it should be noted that each specific technical feature described in the above-mentioned specific embodiments can be combined in any suitable manner unless there is any contradiction. In order to avoid unnecessary repetition, the present application will not further explain the various possible combinations.

In addition, any combination of various embodiments of the present application can also be carried out, as long as they do not violate the idea of the present application, and they should also be regarded as the contents disclosed in the present application.

## Claims

1. An olefin conversion catalyst, comprising the following components, in parts by mass:
a) 50-90 parts, preferably 55-80 parts of a molecular sieve with a structure of twelve-membered ring and above;
b) calculated as oxide, 0.1-10 parts, preferably 1.0-8.0 parts of an added component selected from Group IA metal elements, Group IIA metal elements, or a combination thereof;
c) calculated as oxide, 0.1-10 parts, preferably 1.0-8.0 parts of a modifying component selected from silicon, germanium, bismuth, tin, boron, gallium or a combination thereof; and
d) 10-49 parts, preferably 15-40 parts of a support component.

2. The catalyst according to claim 1, wherein the molecular sieve is selected from Y, β, MCM-22 , MCM-56, SAPO-5, SAPO-37, SAPO-40, RZM- 3 or a combination thereof;
preferably, the molecular sieve is selected from Y, β, MCM-56, MCM-22, SAPO-5, SAPO-37, or a combination thereof;
further preferably, the SiO₂/Al₂O₃ molar ratio of the molecular sieve is 2-60, preferably 5-30.

3. The catalyst according to claim 1 or 2, wherein, as **characterized by** BET nitrogen adsorption and desorption method, the catalyst has the following pore distribution: the pore volume of micropores with diameters of greater than 0.5nm and less than 2.0nm accounts for 4-28%, preferably 5-22% of the total pore volume, the pore volume of mesopores with a diameter of 2-50 nm accounts for 6-50%, preferably 10-40% of the total pore volume.

4. The catalyst according to claim 3, wherein the ratio of the pore volume of the mesopores to the pore volume of the micropores of the catalyst is 0.5-8.0, preferably 0.8-4.5.

5. The catalyst according to claim 3 or 4, wherein the ratio of the mass of the added component calculated as oxide to the product of the mass of the molecular sieve and the ratio of the mesopore volume of the catalyst to the total pore volume of the catalyst is not greater than 0.50, preferably 0.08-0.35.

6. The catalyst according to any one of claims 1 to 5, wherein the support component is selected from or derived from alumina, alumina-containing clay, silica, or a combination thereof, preferably selected from or derived from alumina, kaolin, attapulgite, bentonite, diatomite, silica, or a combination thereof, more preferably alumina.

7. The catalyst according to any one of claims 1 to 6, wherein the added component is selected from calcium, magnesium, potassium, sodium or a combination thereof, preferably magnesium.

8. The catalyst according to any one of claims 1 to 7, wherein the modifying component is a combination of silicon with at least one selected from tin, bismuth, germanium, gallium and boron, preferably a combination of silicon and tin;
preferably, the mass ratio of silicon to non-silicon modifying components is 0.1-10.0 : 1, preferably 1.5-5.0 : 1, calculated as oxide.

9. A method for preparing the olefin conversion catalyst according to any one of claims 1 to 8, comprising the following steps :
(1) shaping a mixture of the molecular sieve, the support and a precursor of the added component, drying and calcining it to obtain a shaped body; and
(2) loading the modifying component on the shaped body, drying and calcining it to obtain a catalyst.

10. The method according to claim 9, wherein:
the precursor of the added component in step (1) is a soluble salt of the added component, preferably a nitrate, a halide, a haloate or a combination thereof;
among the precursors of the modifying component described in step (2), the precursor of silicon is organic silicon; the precursors of germanium, bismuth, gallium and tin are their soluble salts, and the precursor of boron is boric acid;
preferably, the precursor of silicon is selected from ethyl orthosilicate, silicone oil, methyl silicate, siloxane monomers with alkyl of carbon number of 1-4, and halosiloxane monomers with alkyl of carbon number of 1-4 or a combination thereof, the silicone oil is preferably selected from phenyl methyl silicone oil, amino silicone oil, hydroxyl silicone oil or a combination thereof.

11. The method according to claim 9 or 10, wherein the loading process in step (2) includes: firstly the shaped body is impregnated and loaded by a precursor of silicon, and then impregnated and loaded by a precursor of other modifying component selected from germanium, bismuth, tin, gallium and boron or a combination thereof.

12. The method according to any one of claims 9-11, wherein drying conditions in step (2) include: drying temperature being 30-200°C, preferably 60-150°C, drying time being 0.1-72 h, preferably 1-48h; calcination conditions include: calcination temperature being 400-650°C, preferably 450-600°C, calcination time being 0.5-8h, preferably 1-5h; the drying and calcination is conducted in an inert atmosphere.

13. A method for converting olefins contained in an aromatic-rich distillate oil, comprising a step of contacting and reacting an aromatic-rich distillate oil containing olefins with the olefin conversion catalyst according to any one of claims 1 to 8.

14. The method according to claim 13, wherein the aromatic-rich distillate oil is selected from a reformate, an isomerization reaction product, an extracted aromatic hydrocarbons mixture, or a combination thereof; the aromatic hydrocarbon contained in the aromatic-rich distillate oil is benzene, toluene, C8 aromatic hydrocarbons, C9 aromatic hydrocarbons or a combination thereof; in the aromatic-rich distillate oil, the olefin content is expressed in terms of bromine index, and the bromine index is 100-2800 mgBr/100g, preferably 300-2000mgBr/100g.

15. The method according to claim 13 or 14, wherein the reaction is carried out under non-hydrogen conditions, and the reaction conditions include: reaction temperature being 130-350°C, preferably 130-260°C, and reaction pressure being 0.5-4.0MPa, preferably 0.5-3.0MPa, liquid hourly volume space velocity being 0.5-35h^{-1,} preferably 0.5-8h⁻¹.
